(19) Europäisches Patentamt
European Patent Office
Office européen des brevets

(11) Publication number: **0 006 347**
**B1**

(12) **EUROPEAN PATENT SPECIFICATION**

(45) Date of publication of patent specification: **25.01.84**

(21) Application number: **79301126.3**

(22) Date of filing: **13.06.79**

(51) Int. Cl.[3]: **A 01 N 43/78,**
C 07 D 277/00,
C 07 D 263/00,
C 07 D 235/00,
A 01 N 43/76, A 01 N 43/52

(54) **Compositions and method for regulating soybean plant growth utilizing substituted benzazolylthioalkanoic acids.**

(30) Priority: **15.06.78 US 915846**

(43) Date of publication of application:
**09.01.80 Bulletin 80/1**

(45) Publication of the grant of the patent:
**25.01.84 Bulletin 84/4**

(84) Designated Contracting States:
**BE DE FR GB LU NL**

(56) References cited:
**DD - A - 126 082**
**FR - A - 970 993**
**FR - A - 2 202 645**
**US - A - 2 468 075**

**US Department of Agriculture; ARS Special Report 22-65 (Jan 1961) Chemical and Engineering News, Vol 45, p. 18-34 (oct 1978)**

(73) Proprietor: **MONSANTO COMPANY**
**Patent Department 800 North Lindbergh Boulevard**
**St. Louis, Missouri 63166 (US)**

(72) Inventor: **D'Amico, John Joseph**
**1037, Darwick Court**
**St. Louis Missouri 63132 (US)**

(74) Representative: **Lunt, John Cooper et al,**
**Monsanto Europe S.A. Patent Department Avenue de Tervuren 270-272 Letter Box No 1**
**B-1150 Brussels (BE)**

**EP 0 006 347 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Compositions and method for regulating soybean plant growth utilizing substituted benzazolylthioalkanoic acids

This invention relates to the use of certain 2-benzothiazolylthioalkanoic acids, 2-benzoxazolylthioalkanoic acids and 2-benzimidazolylthioalkanoic acids and derivatives thereof to regulate the growth of leguminous plants.

It is known in the art that certain cyanoalkylthiobenzimidazoles are useful as intermediates in the preparation of certain amidoxime substituted-alkylthiobenzimidazoles. These latter compounds are useful as anti-inflammatories. French Patent No. 2,232,315 describes the preparation of the cyanoalkylthiobenzimidazole intermediate compounds and their use in a process to prepare other benzimidazole derivatives which are useful as anti-inflammatories.

U.S. Patent No. 4,006,154, issued February 1, 1977, describes compounds having the formula, $R_2SCH_2CN$, which are useful in the preparation of certain heterocyclic substituted thio and sulfonyl glyoxylonitrileoxime phosphates and phosphonates, these latter compounds having utility as insecticides. $R_2$ is equal to among other groups, benzothiazole, and benzimidazole.

U.S. Patent No. 2,535,876, issued December 26, 1950, describes compounds having the formula

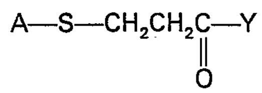

where Y is hydroxyl, alkoxy or $NH_2$ and where A is a nitrogen containing heterocyclic radical. Specifically disclosed is, beta-(2-thio-benzothiazyl)-propionic acid. This patent describes the use of said compounds in a method of plant growth regulation, particularly to promote rooting for purposes of plant propagation and blossom abscission to control fruit set in fruit trees.

This invention relates to the use of certain 2-benzothiazolylthioalkanoic acids, 2-benzoxaolylthioalkanoic acids and 2-benzimidazolylthioalkanoic acids and derivatives thereof to regulate the growth of leguminous plants. More specifically, this invention relates to a method of altering the leaf canopy of soybean plants, which comprises applying to the plant locus a non-phytotoxic effective amount of a compound, characterized in that the compound has the general formula

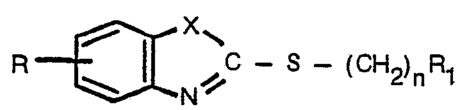

wherein

(a) $R_1$ is COOM where M is H or an agriculturally acceptable cation and n is 1 (i) R is H or 5-Cl and X is S, or (ii) R is H and X is NH:

(b) $R_1$ is $COOC_2H_5$, R is H and n is 1; and X is S or NH:

(c) $R_1$ is $COOCH_3$, R is H and n is 1; and X is O or NH:

(d) $R_1$ is $CONH_2$, n is 1 and (i) R is H or 5-Cl and X is S, or (ii) R is H and X is O:

(e) $R_1$ is $CONHCH_3$, R is H, n is 1 and X is NH: or

(f) $R_1$ is CN and (i) R is H, n is 1 and X is O, or (ii) R is H or 5-Cl, X is S and n is 3.

The invention also provides a composition for altering the leaf canopy of soybean plants which comprises, on the basis of 100 parts by weight, from 5 to 95 parts by weight of a compound having the above general formula, the remaining parts being comprised of one or more suitable adjuvants, carriers and/or diluents.

The term "agriculturally acceptable cation" is understood to mean a cation normally used to form the salt of the free acid, and includes but is not limited to, alkali metal, alkaline earth, ammonium and substituted ammonium cations.

The compounds useful in the present invention are (2-benzothiazolylthio)acetic acid, (5-chloro-2-benzothiazolylthio)acetic acid and (2-benzimidazolylthio)acetic acid and the salts of these acids with agriculturally acceptable cations; ethyl (2-benzothiazolylthio)acetate; ethyl (2-benzimidazolylthio)acetate; methyl (2-benzoxazolylthio)acetate; methyl (2-benzimidazolylthio)acetate; (2-benzothiazolylthio)acetamide; (5-chloro-2-benzothiazolylthio)acetamide; (2-benzoxazolylthio)acetamide; N-methyl-(2-benzimidazolylthio)acetamide; (2-benzoxazolylthio)acetonitrile; 4-(2-benzothiazolylthio)butyronitrile; and 4-(5-chloro-2-benzothiazolylthio)butyronitrile.

It is to be understood that alteration of leaf canopy may occur in conjunction with other responses, but may occur separately. For example, depending upon various factors realized by those skilled in the art to effect activity, the data illustrated below demonstrates that the compounds of the present invention alter the leaf morphology even though the plants are not reduced in stature.

Alteration of the leaf morphology of leguminous plants is important because leguminous plants have canopies that effectively inhibit sunlight from reaching the lower leaves. For example, only about 50% of a soybean plant's leaves intercept light for photosynthesis. Approximately 85% of the light is

absorbed by the outer layer of leaves. Many researchers feel that by altering the morphology of the leaves such that the canopy is altered, light may fall more deeply into the canopy, and yields could be increased. Weber, in "Field Crop Abstracts", Volume 21, No. 4, pages 313—317, states that "greater light penetration, resulting in greater amount of the [soybean] plant canopy having a light intensity above 150 f.c., generally led to higher seed yields." Johnson et al, in "Crop Science", Volume 9, pages 577—581, states that "adding light increased the yields of bottom, middle and top canopy positions of [soybean] plants 30, 20 and 2%, rspectively." Thus, it would be highly beneficial if a method was found whereby the leaves of such plants could be altered such that a greater number of leaves could be illuminated.

The alteration of the leaf canopy of soybeans in accordance with the instant invention does not include the total inhibition or the killing of such plants. It is contemplated herein to employ only such amounts of the compounds as will alter the leaf canopy of the soybean plants. This amount may be applied to the soybeans in sequence at various stages of the plants' development. As may be expected, and as is apparent to those skilled in the art, the required amount will vary, not only with the compound selected, but also with stage of development of the plant, the plant growth medium and whether a permanent or transitory effect is sought.

The acids of the foregoing formula (I) may be prepared by reaction of the appropriate 2-mercaptobenzothiazole or 2-mercaptobenzimidazole with bromoacetic acid and sodium hydroxide. For sake of clarity, the above reaction procedure is illustrated by the reaction scheme below:

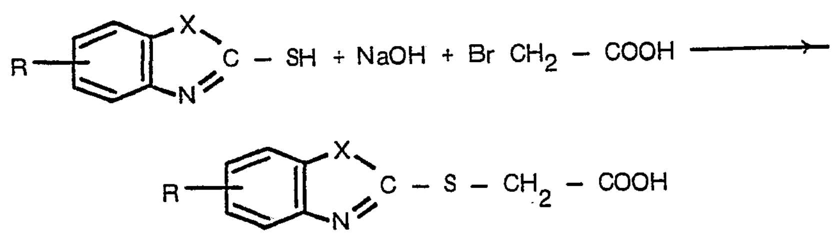

The compounds identified in Table I, below, were prepared in accordance with the above reaction scheme as follows.

To a stirred solution containing 35 g (0.25 mole) of bromoacetic acid in 100 ml. of water, 18.6 g (0.125 mole) of potassium carbonate is added in small portions until a pH of 8 is obtained. This solution is added to a stirred solution containing 0.25 moles of the appropriate mercaptan, 20 g (0.25 mole) of 50% aqueous sodium hydroxide and 200 ml. of water. The stirred solution is heated at reflux for 6 hours. After cooling to 25°C., 30 g (0.3 mole) of concentrated hydrochloric acid in 500 ml. of water is added dropwise until pH of 2 is obtained. After stirring at 25—30°C. for 15 minutes, the solid is collected by filtration, washed with water until neutral to litmus and air-dried at 50°C. The data are summarized in Table I.

TABLE I

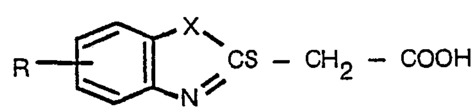

| Compound Number | R | X | m.p. °C. | Percent Yield | Percent N Calc'd. | Percent N Found | Percent S Calc'd. | Percent S Found |
|---|---|---|---|---|---|---|---|---|
| 1 | H | S | 153—5 | 99 | 6.22 | 6.10 | 28.47 | 28.57 |
| 2 | 5-Cl | S | 168—170 | 95 | 5.39 | 5.26 | 24.69 | 24.52 |
| 3 | H | NH | 204—6 | 93 | 13.45 | 13.44 | 15.40 | 15.30 |

Esters are prepared in accordance with the reaction scheme below by reacting the appropriate 2-mercaptobenzoxazole, 2-mercaptobenzothiazole or 2-mercaptobenzimidazole with a bromoacetic ester under basic conditions as follows:

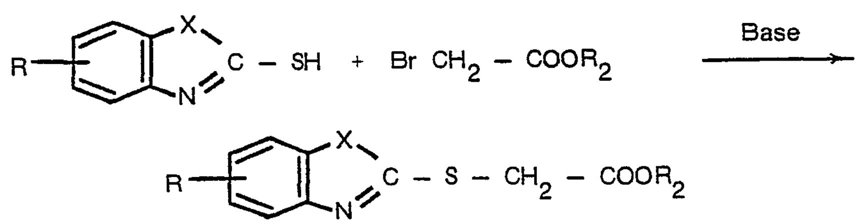

The compounds identified in Tables II and III, below, were prepared in accordance with the above reaction scheme as follows.

To a stirred solution containing 0.1 mole of the appropriate mercaptan, 6.6 g (0.1 mole) of 85% potassium hydroxide, and 200 ml. of ethanol, 17.8 g (0.1 mole) of 94% ethyl bromoacetate is added in one portion. The stirred reaction mixture is heated at reflux for 24 hours. After cooling to 5°C., 800 grams of ice water is added and stirring continued at 0—10°C. for one hour. The solid is collected by filtration, washed with cold water until neutral to litmus and air-dried at 25—30°C. The data are summarized in Table II.

TABLE II

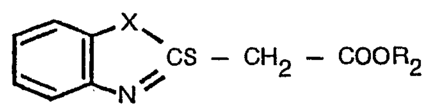

| Compound No. | X | $R_2$ | m.p. °C. | Percent Yield | Percent C Calc'd. | Percent C Found | Percent H Calc'd. | Percent H Found | Percent N Calc'd. | Percent N Found | Percent S Calc'd. | Percent S Found |
|---|---|---|---|---|---|---|---|---|---|---|---|---|
| 4 | S | $-C_2H_5$ | 39—40 | 95 | 52.15 | 52.10 | 4.38 | 4.38 | 5.53 | 5.56 | 25.31 | 25.36 |
| 5 | NH | $-C_2H_5$ | 92—3[(a)] | 85 | 55.91 | 55.73 | 5.12 | 5.18 | 11.86 | 11.77 | 13.57 | 13.45 |

(a) Recrystallization from heptane-ethyl alcohol.

To a stirred solution containing (0.1 mole) of the appropriate mercaptan, 21.6 g (0.1 mole) of 25% sodium methylate in methyl alcohol and 200 ml. of methyl alcohol, 16.8 g (0.1 mole) of methyl bromoacetate is added in one portion. The stirred reaction mixture is heated at reflux for 24 hours. The rest of the procedure is identical as that described above to prepare the ethyl esters. The data are summarized in Table III.

TABLE III

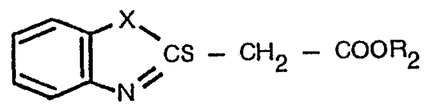

| Compound No. | X | $R_2$ | m.p. °C. | Percent Yield | Percent C Calc'd. | Percent C Found | Percent H Calc'd. | Percent H Found | Percent N Calc'd. | Percent N Found | Percent S Calc'd. | Percent S Found |
|---|---|---|---|---|---|---|---|---|---|---|---|---|
| 6 | O | $—CH_3$ | 53—4 | 81 | 53.80 | 53.66 | 4.06 | 4.10 | 6.27 | 6.31 | 14.36 | 14.32 |
| 7 | NH | $—CH_3$ | 110—11[a] | 87 | 54.04 | 54.16 | 4.54 | 4.61 | 12.60 | 12.65 | 14.43 | 14.40 |

[a] Recrystallization from heptane-ethyl alcohol.

Amides of the foregoing formula are prepared by reaction of the appropriate 2-mercaptobenzoxazole, 2-mercaptobenzothiazole or 2-mercaptobenzimidazole with the appropriate chlorinated amide and potassium hydroxide in accordance with the following reaction scheme:

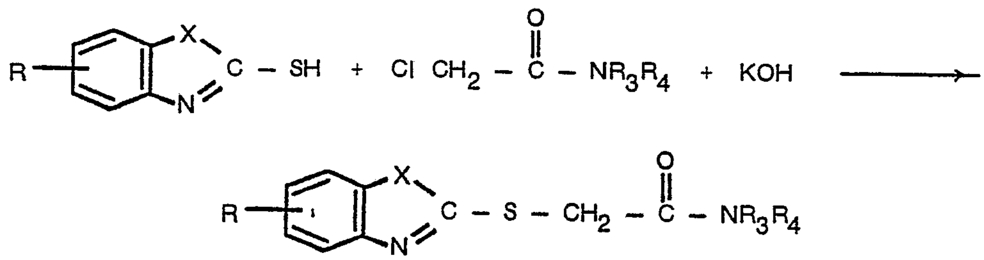

The compounds identified in Table IV, below, have been prepared in accordance with the above procedure as follows.

To a stirred solution containing 0.2 mole of the appropriate mercaptan, 13.2 g (0.2 mole) of 85% potassium hydroxide, 300 ml. of acetone and 20 ml of water, 0.2 mole of 2-chloroacetamide or 2-chloro N-methylacetamide is added in one portion. The stirred reaction mixture is heated at reflux for eight hours and at 25—30°C. for 18 hours. After the addition of 800 ml. of water, the reaction mixture is stirred at 25—30°C. for 30 minutes. The solid is collected by filtration, washed with water until neutral to litmus and air-dried at 25—30°C. The data are summarized in Table IV.

TABLE IV

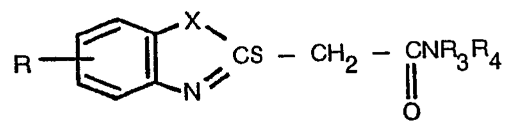

| Compound No. | R | X | $R_3$ | $R_4$ | m.p. °C. | Percent Yield | Percent C Calc'd. | Percent C Found | Percent H Calc'd. | Percent H Found | Percent N Calc'd. | Percent N Found | Percent S Calc'd. | Percent S Found |
|---|---|---|---|---|---|---|---|---|---|---|---|---|---|---|
| 8 | H | S | H | —H | 147—8[a] | 83 | — | — | — | — | 12.49 | 12.10 | — | — |
| 9 | 5-Cl | S | H | —H | 187—8[b] | 91 | 41.78 | 41.62 | 2.73 | 2.69 | — | — | — | — |
| 10 | H | O | H | —H | 163—4[a] | 80 | — | — | — | — | 13.45 | 13.59 | 15.40 | 15.58 |
| 11 | H | NH | H | —$CH_3$ | 178—9[a] | 69 | 54.28 | 54.25 | 5.01 | 5.07 | 18.99 | 18.96 | 14.49 | 14.40 |

[a] Recrystallization from isopropyl alcohol.
[b] Recrystallization from methyl alcohol.

Nitriles of the foregoing formula are prepared by reacting the appropriate 2-mercaptobenzoxazole, or 2-mercaptobenzothiazole with potassium hydroxide and chloroacetonitrile, or chlorobutyronitrile in accordance with the following reaction scheme:

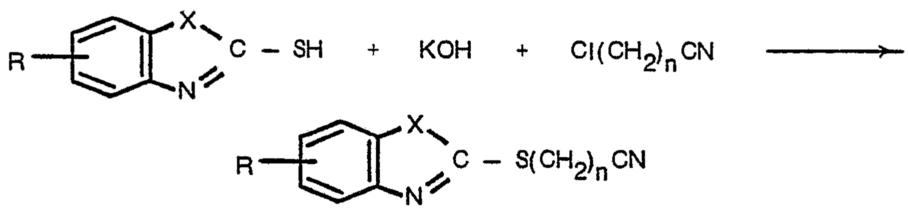

In accordance with the above procedure, 2-benzoxazolethioacetonitrile (Compound No. 12) has been prepared by adding 8.4 g (0.11 mole) of chloroacetonitrile in one portion to a stirred solution containing 0.1 mole of 2-mercaptobenzoxazole, 6.6 g (0.1 moles) of 85% potassium hydroxide, 200 ml. of acetone and 10 ml. of water. An exothermic reaction set in causing a temperature rise from 25 to 50°C. The stirred reaction mixture is heated at reflux for six hours. After cooling to 5°C., 800 grams of ice water is added and stirring continued at 0—10°C. for 30 minutes. The solid is collected by filtration, washed with water until neutral to litmus and air-dried at 25—30°C. Compound No. 12 having the structure.

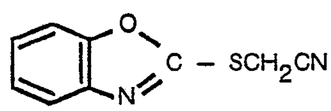

was obtained in 92% yield, m.p. 97—98°C. from isopropyl alcohol.

Anal. Calc'd: C, 56.83; H, 3.18; N, 14.73; S, 16.86.
Found: C, 56.80; H, 3.19; N, 14.70; S, 16.86.

In a similar manner, the compounds identified in Table V have been prepared.

To a stirred solution containing 0.2 mole of the appropriate mercaptan, 13.2 g (0.2 mole) of 85% potassium hydroxide, 200 ml. of dimethyl formamide and 20 ml. of water, 20.7 grams (0.2 mole) of 4-chlorobutyronitrile is added in one portion. The stirred reaction mixture is heated at 90—100°C. for six hours and at 25—30°C. for 18 hours.

To prepare Compound No. 13, 500 ml. of water and 600 ml. of ethyl ether are added and stirring continued at 25—30°C. for 15 minutes. The separated ether layer is washed with water until neutral to litmus and dried over sodium sulfate. The ether is removed in vacuo at a maximum temperature of 80—90°C. at 1—2 mm.

To prepare Compound No. 14, 800 grams of ice water is added and stirring continued at 10—20°C. for 30 minutes. The solid is collected by filtration, washed with water until neutral to litmus and air-dried at 25—30°C. The data are summarized in Table V.

TABLE V

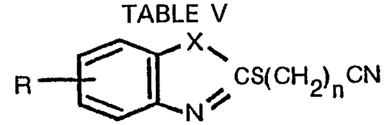

| Compound No. | R | X | n | m.p. °C. | Percent Yield | $N_D^{25}$ | Percent N Calc'd. | Percent N Found | Percent S Calc'd. | Percent S Found | Percent Cl Calc'd. | Percent Cl Found |
|---|---|---|---|---|---|---|---|---|---|---|---|---|
| 13 | H | S | 3 | amber liquid | 90 | 1.6405 | 11.96 | 11.85 | 27.37 | 27.16 | — | — |
| 14 | 5-Cl | S | 3 | 74—5[a] | 98 | — | 10.42 | 10.52 | 23.86 | 24.05 | 13.19 | 13.09 |

[a] Recrystallization from heptane-isopropyl alcohol.

To illustrate the variety of regulatory responses observed, the compounds of the invention were tested in accordance with the following procedure.

A number of soybean plants are grown from seeds in plastic pots in the greenhouse for a period of one week at which time the plants are thinned to one plant per pot. When the second trifoliate leaf is fully expanded, the plants are treated with a solution of the active ingredient in acetone and water. Aqueous Tween® 20 is used as a surfactant.

When the fifth trifoliate leaf of the control is fully expanded, the treated plants are compared with the non-treated control plants and the observations recorded.

The following observations were made when soybeans were treated at the indicated rates with the compounds of the foregoing formula.

| Compound Number | Rate (kg/ha) | Observations |
|---|---|---|
| 1 | 2.8 | Stature reduction, inhibition of dry weight, slight leaf burn, leaf distortion, leaf alteration, leaf inhibition, altered canopy. |
| | 0.56 | Leaf alteration, leaf inhibition, altered canopy. |
| | 0.112 | No response. |
| 4 | 2.8 | Stature reduction, inhibition of dry weight, slight leaf burn, leaf alteration of old and new growth, leaf inhibition, altered canopy. |
| | 0.56 | Stature reduction, inhibition of dry weight, leaf alteration of old and new growth, leaf inhibition, altered canopy. |
| | 0.112 | Leaf alteration of new growth. |
| 8 | 2.8 | Stature reduction, inhibition of dry weight, leaf alteration, slight leaf burn, leaf distortion, leaf inhibition, altered canopy. |
| | 0.56 | Leaf alteration, leaf inhibition, altered canopy. |
| | 0.112 | No response. |
| 3 | 2.8 | Stature reduction, leaf alteration of old and new growth, leaf inhibition, altered canopy. |
| | 0.56 | Stimulation of dry weight. |
| | 0.112 | No response. |
| 14 | 2.8 | Stature reduction, inhibition of dry weight, moderate leaf burn, chlorosis, leaf alteration, altered canopy. |
| | 0.56 | Inhibition of dry weight, slight leaf burn, chlorosis. |
| | 0.112 | Inhibition of dry weight. |
| 6 | 2.8 | Stature reduction, inhibition of dry weight, slight leaf burn, leaf distortion, leaf inhibition, altered canopy, inhibition of apical development. |
| | 0.56 | No response. |
| | 0.112 | No response. |

| Compound Number | Rate (kg/ha) | Observations |
|---|---|---|
| 7 | 2.8 | Stature reduction, inhibition of dry weight, slight leaf burn, leaf alteration, leaf inhibition, altered canopy, inhibition of apical development. |
| | 0.56 | No response. |
| | 0.112 | No response. |
| 5 | 2.8 | Stature reduction, inhibition of dry weight, leaf alteration of old and new growth, leaf inhibition, altered canopy. |
| | 0.56 | Inhibition of dry weight, leaf alteration of old and new growth. |
| | 0.112 | Leaf alteration of old and new growth. |
| 11 | 2.8 | Stature reduction, inhibition of dry weight, slight leaf burn, leaf distortion, leaf alteration of old and new growth, leaf inhibition. |
| | 0.56 | Inhibition of dry weight, leaf alteration of old and new growth, altered canopy. |
| | 0.112 | Inhibition of dry weight, leaf alteration of old and new growth. |
| 13 | 2.8 | Stature reduction, inhibition of dry weight, slight leaf burn, leaf distortion, leaf alteration, altered canopy. |
| | 0.56 | No response. |
| | 0.112 | No response. |
| 9 | 2.8 | Inhibition of dry weight. |
| | 0.56 | Inhibition of dry weight. |
| | 0.112 | Inhibition of dry weight. |

The compounds of the invention were further tested as follows.

A number of soybean plants are grown from seeds in aluminum pans in the greenhouse for a period of approximately 1 week to the primary leaf stage. The plants are thinned to three uniform plants in each pan and the height of each plant in the pan is measured to the terminal bud and the average height is noted. One pan containing three soybean plants is used for each chemical treated and three pans are not treated and used as a control. The composition formulated with acetone and Tween® 20 as a surfactant is then applied to the pan of growing plants by overhead spray at a rate equivalent to the desired rate of active ingredient per acre. The treated pans, along with the control pans, are maintained in a greenhouse and watered from below on a sand bench and fertilized with a uniform portion of a water-soluble balanced fertilizer.

Two weeks after application of the chemical, the average height of the soybean plants in the treated pan is again measured as above and the difference in the average height before and 2 weeks after application represents the increase in the development of the treated pans. This development in growth of the treated plants is compared to the average increase in growth of the plants in the control pans during the same period of time. A variation of 25% or more in the development of at least two-thirds of the treated plants when compared to the development of the control plants demonstrates that the chemical is an effective plant regulant. Thus, a chemical is considered active when the treated plants manifest a decrease in growth of at least 25% less than that of the control plants, i.e., stature reduction, or an increase in growth in excess of 25% of that of the control plants, i.e., growth stimulation.

The following results and observations were made when the benzothiazolines of the invention were utilized as the active ingredient at several rates.

| Compound Number | Rate (kg/ha) | Observations |
|---|---|---|
| 8 | 6.72 | Stature reduction, stem distortion, slight leaf burn, altered canopy, stimulation of axillary buds. |
| | 3.36 | Stature reduction, leaf alteration, altered canopy, stimulation of axillary buds. |
| | 3.36 | Stature reduction, stem distortion, altered canopy, stimulation of axillary buds. |
| | 1.34 | Leaf alteration. |
| | 0.67 | Leaf alteration. |
| 10 | 6.72 | Chlorosis, leaf alteration, altered canopy, stimulation of axillary buds. |
| | 6.72 | Chlorosis, leaf alteration, altered canopy, stimulation of axillary buds, slight leaf burn. |
| | 3.36 | Leaf alteration, altered canopy, stimulation of axillary buds. |
| | 1.34 | Stimulation of axillary buds. |
| 2 | 6.72 | Stature reduction, slight leaf burn, defoliation, altered canopy. |
| | 3.36 | Stature reduction, slight leaf burn, defoliation, leaf inhibition. |
| | 1.34 | Stature reduction, slight leaf burn, stimulation of axillary buds. |
| | 0.67 | Stature reduction, altered canopy, stimulation of axillary buds. |
| | 0.67 | Stature reduction, altered canopy, stimulation of axillary buds, slight leaf burn. |
| | 0.36 | Altered canopy, slight leaf burn. |
| | 0.112 | No response. |
| 12 | 6.72 | Altered canopy, leaf alteration stimulation of axillary buds. |
| | 6.72 | Stature reduction, slight leaf burn, inhibition of apical development, stimulation of axillary buds. |
| | 3.36 | Altered canopy, leaf alteration, stimulation of axillary buds. |
| | 1.34 | Leaf alteration. |
| 13 | 6.72 | Stature reduction, slight leaf burn, defoliation. |
| | 3.36 | Stature reduction, slight leaf burn, defoliation. |
| | 1.34 | Stimulation of axillary buds, slight leaf burn. |

| Compound Number | Rate (kg/ha) | Observations |
|---|---|---|
| 9 | 6.72 | Altered canopy, stimulation of axillary buds. |
| | 6.72 | Leaf distortion, slight leaf burn, stimulation of axillary buds. |
| | 3.36 | Stimulation of axillary buds. |
| | 1.34 | No response. |

Further tests were conducted as follows.

Individual soybean plants are grown from seed in 6-inch pots containing a good grade of top soil. Two pots of 6-week old plants (5—6 trifoliate stage) are used for each application of the chemical. An overhead spray of an aqueous composition of the chemical is applied to the pots at an equivalent rate as indicated below. Two to four sets of plants which received no chemical application are included and serve as controls. All of the pots are maintained under good growing conditions and are watered and are fertilized with a uniform amount of water-soluble balanced fertilizer. Two weeks after the application of the chemical, the growth responses of the treated plants are compared with that of the control plants. The total height of the plant is measured to the tip of the terminal bud. A variation of 15% in the average total height of the treated plants, when compared to the average total height of the control plants, demonstrate that the chemical is an effective plant growth regulator. Observations made utilizing this procedure are summarized as follows.

| Compound Number | Rate (kg/ha) | Observations |
|---|---|---|
| 8 | 2.8 | Stature reduction, leaf distortion. |
| | 1.12 | Stature reduction, leaf distortion, leaf alteration. |
| 12 | 2.8 | Leaf distortion, leaf alteration, delayed pod set, inhibited pod set. |
| | 1.12 | Stature reduction, leaf alteration. |
| | 0.56 | No response. |

From the above data, it can be seen that the compounds of the present invention are especially effective in reducing the stature of the soybean plant as well as altering the leaf morphology.

In practicing the method of this invention, the active ingredient can be used alone or in combination with a material referred to in the art as an adjuvant in liquid or solid form. The compositions of this invention are prepared by admixing the active ingredient with an adjuvant including diluents, extenders, carriers and conditioning agents to provide compositions in the form of finely-divided particulate solids, granules, pellets, wettable powders, dusts, solutions and aqueous dispersions or emulsions. Thus, the active ingredient can be used with an adjuvant such as a finely-divided particulate solid, a solvent liquid of organic origin, water, a wetting agent, dispersing agent, or emulsifying agent or any suitable combination of these.

When applying the active ingredient to soybean plants, useful finely-divided solid carriers and extenders include, for example, the talcs, clays, pumice, silica, diatomaceous earth, quartz, Fullers earth, sulfur, powdered cork, powdered wood, walnut flour, chalk, tobacco dust, charcoal and the like. Typical liquid diluents useful in applying the active ingredient include, for example, Stoddard solvent, acetone, alcohols, glycols, ethyl acetate, benzene and the like. Such compositions, particularly liquids and wettable powders, usually contain as a conditioning agent one or more surface-active agents in amounts sufficient to render a given composition readily dispersible in water or in oil. By the term "surface-active agent", it is understood that wetting agents, dispersing agents, suspending agents and emulsifying agents are included therein. Such surface-active agents are well known and reference is made to U.S. Patent No. 2,547,724, columns 3 and 4, for detailed examples of the same.

Compositions of this invention generally contain on the basis of 100 parts from 5 to 95 parts active ingredient, 1 to 50 parts surface-active agent and 4 to 94 parts solvent, all parts being by weight based on the total weight of the composition.

The required modification of soybean plants may be achieved by applying the above-described

compounds to the plant locus. The term "plant locus" is understood herein to include the plant growing medium, such as the soil, as well as the seeds, emerging seedlings, roots, stems, leaves, flowers, fruits or other plant parts.

The application of liquid and particulate solid compositions of the active ingredient can be carried out by conventional techniques utilizing, for example, spreaders, power dusters, boom and hand sprayers and spray dusters. The compositions can also be applied from airplanes as a dust or spray. If desired, application of the compositions of the invention can be accomplished by incorporating the compositions in the soil or other media in the area where modification of the plants is desired.

In selecting the appropriate non-toxic rate of application of the active ingredient. It will be recognized that precise rates will also be dependent upon the mode of application, such as soil incorporation, band application, pre-plant seed treatment, result desired and various other factors known to those skilled in the art. In applications to the soil habitat of germinant seeds, emerging seedlings, and established vegetation, the active ingredients are applied in amounts of from 0.056 to 22.4 kilos/hectare. Foliar application is particularly advantageous and is preferred especially from 0.112 to 6.7 kilos/hectare.

Certain compounds mentioned in this specification are new. Therefore a further aspect of the invention are compounds having the formula

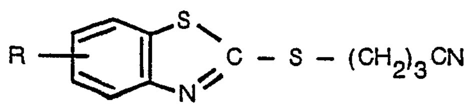

wherein R is hydrogen or 5-Cl. The preferred compound has the above formula wherein R is hydrogen.

**Claims**

1. A method of altering the leaf canopy of soybean plants, which comprises applying to the plant locus a non-phytotoxic effective amount of a compound, characterized in that the compound has the general formula

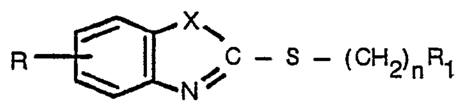

wherein

(a) $R_1$ is COOM where M is H or an agriculturally acceptable cation and n is 1 (i) R is H or 5-Cl and X is S, or (ii) R is H and X is NH:

(b) $R_1$ is $COOC_2H_5$, R is H and n is 1; and X is S or NH:

(c) $R_1$ is $COOCH_3$, R is H and n is 1; and X is O or NH:

(d) $R_1$ is $CONH_2$, n is 1 and (i) R is H or 5-Cl and X is S, or (ii) R is H and X is O:

(e) $R_1$ is $CONHCH_3$, R is H, n is 1 and X is NH: or

(f) $R_1$ is CN and (i) R is H, n is 1 and X is O, or (ii) R is H or 5-Cl, X is S and n is 3.

2. A composition for altering the leaf canopy of soybean plants which comprises, on the basis of 100 parts, from 5 to 95 parts by weight of a compound, the remaining parts being comprised of one or more suitable adjuvants, carriers and/or diluents, characterized in that compound has the general formula

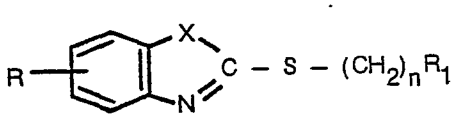

wherein

(a) $R_1$ is COOM where M is H or an agriculturally acceptable cation and n is 1 (i) R is H or 5-Cl and X is S, or (ii) R is H and X is NH:

(b) $R_1$ is $COOC_2H_5$, R is H and n is 1; and X is S or NH:

(c) $R_1$ is $COOCH_3$, R is H and n is 1; and X is O or NH:

(d) $R_1$ is $CONH_2$, n is 1 and (i) R is H or 5-Cl and X is S, or (ii) R is H and X is O:

(e) $R_1$ is $CONHCH_3$, R is H, n is 1 and X is NH: or

(f) $R_1$ is CN and (i) R is H, n is 1 and X is O, or (ii) R is H or 5-Cl, X is S and n is 3.

3. A composition according to Claim 2 wherein $R_1$ is CN, n is 3, R is hydrogen and X is sulfur.

4. A compound having the formula

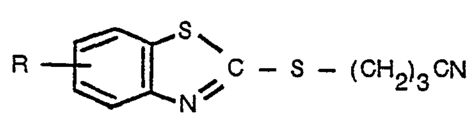

wherein R is hydrogen or 5-Cl.

5. A compound according to Claim 4 wherein R is hydrogen.

**Revendications**

1. Procédé pour modifier la voûte des feuilles de plantes du genre soja, qui consiste à appliquer au lieu des plantes une quantité efficace non phytoxique d'un composé, caractérisé en ce que le composé a la formule générale

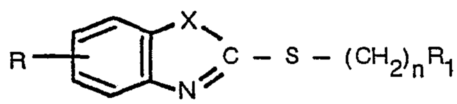

où

(a) $R_1$ est COOM où M est H ou un cation acceptable en agriculture et n est 1 (i) R est H ou 5-Cl et X est S, ou (ii) R est H et X est NH;

(b) $R_1$ est $COOC_2H_5$, R est H et n est 1; et X est S ou NH;

(c) $R_1$ est $COOCH_3$, R est H et n est 1; et X est O ou NH;

(d) $R_1$ est $CONH_2$, n est 1 et (i) R est H ou 5-Cl et X est S, ou (ii) R est H et X est O;

(e) $R_1$ est $CONHCH_3$, R est H, n est 1 et X est NH; ou

(f) $R_1$ est CN et (i) R est H, n est 1 et X est O, ou (ii) R est H ou 5-Cl, X est S et n est 3.

2. Composition pour modifier la voûte des feuilles de plantes du genre soja, qui comprend, sur la base de 100 parties, 5 à 95 parties en poids d'un composé, les parties restantes étant formées d'un ou de plusieurs adjuvants, supports et/ou diluants convenables, caractérisée en ce que le composé a la formule générale

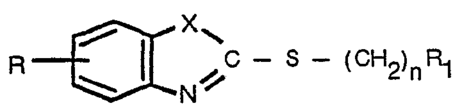

où

(a) $R_1$ est COOM où M est H ou un cation acceptable en agriculture et n est 1, et (i) R est H ou 5-Cl et X est S, ou (ii) R est H et X est NH:

(b) $R_1$ est $COOC_2H_5$, R est H et n est 1; et X est S ou NH;

(c) $R_1$ est $COOCH_3$, R est H et n est 1; et X est O ou NH;

(d) $R_1$ est $CONH_2$, n est 1 et (i) R est H ou 5-Cl et X est S, ou (ii) R est H et X est O:

(e) $R_1$ est $CONHCH_3$, R est H, n est 1 et X est NH; ou

(f) $R_1$ est CN et (i) R est H, n est 1 et X est O, ou (ii) R est H ou 5-Cl, X est S et n est 3.

3. Composition selon la revendication 2, dans laquelle $R_1$ est CN, n est 3, R est l'hydrogène et X est le soufre.

4. Composé ayant la formule

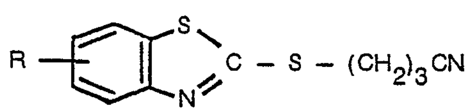

où R est l'hydrogène ou 5-Cl.

5. Composé selon la revendication 4, dans lequel R est l'hydrogène.

**Patentansprüche**

1. Verfahren zur Änderung des Blätterdaches von Sojabohnenpflanzen, wobei auf den Pflanzenort eine nicht-phytotoxische wirksame Menge einer Verbindung aufgebracht wird, dadurch gekennzeichnet, daß die Verbindung die allgemeine Formel

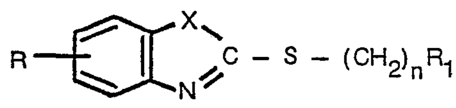

hat, worin

(a) $R_1$ für COOM steht, wobei M ein Wasserstoffatom oder ein landwirtschaftlich verträgliches Kation ist und n die Zahl 1 ist, (i) R für H oder 5-Cl steht und X gleich S ist, oder (ii) R für H steht und X gleich NH ist;

(b) $R_1$ für $COOC_2H_5$ steht, R gleich H ist und n die Zahl 1 bedeutet; und X für S oder NH steht;

(c) $R_1$ für $COOCH_3$ steht, R gleich H ist und n die Zahl 1 bedeutet; und X für O oder NH steht;

(d) $R_1$ für $CONH_2$ steht, n gleich 1 ist und (i) R für H oder 5-Cl steht und X gleich S ist, oder (ii) R für H und X für O steht;

(e) $R_1$ für $CONHCH_3$ steht, R gleich H ist, n die Zahl 1 bedeutet und X für NH steht; oder

(f) $R_1$ für CN steht und (i) R gleich H ist, n die Zahl 1 bedeutet und X für O steht, oder (ii) R für H oder 5-Cl steht, X gleich S ist und n die Zahl 3 bedeutet.

2. Zusammensetzung zur Änderung des Blätterdaches von Sojabohnenpflanzen, welche auf der Basis von 100 Teilen, 5 bis 95 Gewichtsteile einer Verbindung enthält, wobei die verbleibenden Teile von einem oder mehreren geeigneten Zusatzstoffen, Trägern und/oder Verdünnungsmitteln eingenommen werden, dadurch gekennzeichnet, daß die Verbindung die allgemeine Formel

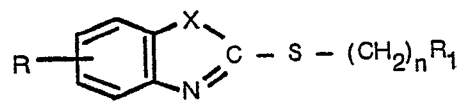

aufweist, worin

(a) $R_1$ für COOM steht, wobei M ein Wasserstoffatom oder ein landwirtschaftlich verträgliches Kation ist und n die Zahl 1 ist, und (i) R für H oder 5-Cl steht und X gleich S ist, oder (ii) R für H steht und X gleich NH ist;

(b) $R_1$ für $COOC_2H_5$ steht, R gleich H ist und n die Zahl 1 bedeutet; und X für S oder NH steht;

(c) $R_1$ für $COOCH_3$ steht, R gleich H ist und n die Zahl 1 bedeutet; und X für O oder NH steht;

(d) $R_1$ für $CONH_2$ steht, n die Zahl 1 ist und (i) R für H oder 5-Cl steht und X gleich S ist, oder (ii) R gleich H und X gleich O ist;

(e) $R_1$ für $CONHCH_3$ steht, R gleich H ist, n die Zahl 1 ist und X für NH steht; oder

(f) $R_1$ für CN steht und (i) R gleich H ist, n die Zahl 1 bedeutet und X gleich O ist, oder (ii) R für H oder 5-Cl steht, X gleich S ist und n die Zahl 3 ist.

3. Zusammensetzung nach Anspruch 2, worin $R_1$ für CN steht, n die Zahl 3 ist, R Wasserstoff bedeutet und X Schwefel darstellt.

4. Verbindung der Formel

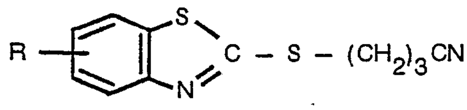

worin R ein Wasserstoffatom oder 5-Cl bedeutet.

5. Verbindung nach Anspruch 4, worin R Wasserstoff ist.